# EUROPEAN PATENT APPLICATION

(11) **EP 2 251 023 A1**
(43) Date of publication of application: **17.11.2010**
(21) Application number: 09719500.2
(22) Date of filing: 26.02.2009
(51) Int. Cl.: A61K 36/18, A01N 43/16, A01N 65/00, A61K 31/353, A61P 3/00, A61P 31/04, C07D 311/32

(54) **ANTI-METHICILLIN-RESISTANT STAPHYLOCOCCUS AUREUS AGENT AND ANTI-VANCOMYCIN-RESISTANT ENTEROCOCCUS AGENT**

(30) Priority: 10.03.2008 JP 2008059783
(71) Applicant: POKKA CORPORATION, Nagoya-shi Aichi 460-8415 (JP)
(72) Inventor: GOTO, Takaki, Kitanagoya-shi Aichi 481-8515 (JP); FUKUMOTO, Syuichi, Kitanagoya-shi Aichi 481-8515 (JP)
(74) Representative: Melin Granberg, Linda
(86) International application number: PCT/JP2009/053587
(87) International publication number: WO 2009/113403

(57) **Abstract**

An anti-methicillin-resistant *Staphylococcus aureus* agent (anti-MRSA agent) and an anti-vancomycin-resistant *Enterococcus* agent (anti-VRE agent) contain as an active ingredient an Oobagi extract extracted from Oobagi with an extraction solvent including at least an organic solvent. Alternatively, the anti-MRSA agent and the anti-VRE agent contain as an active ingredient at least one selected from nymphaeol-A, nymphaeol-B, and nymphaeol-C.

## Description

### TECHNICAL FIELD

The present invention relates to an anti-methicillin-resistant *Staphylococcus aureus* agent and an anti-vancomycin-resistant *Enterococcus* agent.

### BACKGROUND ART

Methicillin-resistant *Staphylococcus aureus* (MRSA) is a multidrug-resistant bacterium that exhibits resistance to β-lactam antibiotics such as cephem antibiotics including penicillin antibiotics, into which methicillin is classified, monobactam antibiotics, and carbapenem antibiotics. Besides β-lactam antibiotics, MRSA is also known to exhibit resistance to, for example, aminoglycoside antibiotics and macrolide antibiotics.

Vancomycin, teicoplanin, arbekacin, and linezolid are used as anti-MRSA drugs. Particularly, the emergence of vancomycin-resistant bacteria has been rare, and thus vancomycin has been perceived as effective in treating an MRSA infection. However, the emergence of vancomycin-resistant *Enterococcus* (VRE) such as *Enterococcus* faecalis and *Enterococcus faecium* has been discovered recently.

It is known that some naturally derived ingredients exhibit antimicrobial activity against MRSA or VRE. For example, Patent Document 1 discloses that a compound extracted from Artemisia gilvescens Miq exhibits an anti-MRSA activity. Also, Patent Document 2 discloses that hinokitiol exhibits an anti-VRE activity.

As described in Patent Document 3, it is known that an extract of *Macaranga tanarius* (Oobagi), which belongs to the genus *Macaranga* of the family *Euphorbiaceae,* has an antimicrobial action. However, the action of the *Macaranga tanarius* extract on MRSA and VRE has not been clarified yet, and also Patent Document 3 does not describe it at all.

Since there is a concern that MRSA and VRE might acquire resistances one after another, to find a novel ingredient that exhibits antimicrobial activity against MRSA or VRE is a crucial task.
Patent Document 1: Japanese Laid-Open Patent Publication No. 2003-201234
Patent Document 2: Japanese Laid-Open Patent Publication No. 2001-131061
Patent Document 3: Japanese Laid-Open Patent Publication No. 2007-45754

### DISCLOSURE OF THE INVENTION

The present invention is based on the fact that the inventors have found, as a result of their intensive studies, that an extract of *Macaranga tanarius* exhibits antimicrobial activity against MRSA and VRE, and an objective thereof is to provide a novel anti-MRSA agent and a novel anti-VRE agent.

In order to achieve the above-mentioned objective, a first aspect of the present invention provides an anti-MRSA agent containing as an active ingredient a *Macaranga tanarius* extract extracted from *Macaranga tanarius* with an extraction solvent including at least an organic solvent.

A second aspect of the present invention provides an anti-MRSA agent containing as an active ingredient at least one selected from nymphaeol-A, nymphaeol-B, and nymphaeol-C.

A third aspect of the present invention provides an anti-VRE agent containing as an active ingredient a *Macaranga tanarius* extract extracted from *Macaranga tanarius* with an extraction solvent including at least an organic solvent.

A fourth aspect of the present invention provides an anti-VRE agent containing as an active ingredient at least one selected from nymphaeol-A, nymphaeol-B, and nymphaeol-C.

### BRIEF DESCRIPTION OF THE DRAWINGS

Fig. 1 is a chromatogram showing the results of high-performance liquid chromatography analysis of an extract of *Macaranga tanarius* according to Example 1; and
Fig. 2 is a chromatogram showing the results of high-performance liquid chromatography analysis of an extract of *Macaranga tanarius* according to Example 2.

### BEST MODE FOR CARRYING OUT THE INVENTION

### First Embodiment

An anti-MRSA agent according to a first embodiment of the present invention will now be described.

An anti-MRSA agent of the present embodiment contains as an active ingredient an Oobagi extract extracted from Oobagi with an extraction solvent including at least an organic solvent.

Oobagi is also called *Macaranga tanarius* and is a dioecious broad-leaved evergreen tree belonging to the genus *Macaranga* of the family *Euphorbiaceae. Macaranga tanarius* grows, for example, in Southeast Asia, such as Okinawa (southern Japan), Taiwan, southern China, the Malay Peninsula, the Philippines, Malaysia, Indonesia, and Thailand, and in northern Australia. *Macaranga tanarius* grows significantly fast compared to other trees and can grow on degraded lands.

All the organs of *Macaranga tanarius* and constituents of each organ can be used as raw material to be subjected to extraction with the extraction solvent. The raw material for extraction may be a single organ of *Macaranga tanarius* or its constituents or may be a mixture of two or more organs of *Macaranga tanarius* or their constituents. In order to enhance the anti-MRSA activity of the resulting *Macaranga tanarius* extract, it is preferred to use the raw material for extraction which includes fruit, seeds, flowers, roots, a trunk, the tip of a stem, a leaf blade, or an exudate (such as wax) of *Macaranga tanarius.* Since the tip of the stem includes a growth point of the stem and a leaf bud and is softer than the leaf blade, an efficient extraction procedure thereof is easy. Furthermore, the occupation ratios of the trunk, the roots, and the leaves to the entire *Macaranga tanarius* are high compared to those of other organs. Therefore, the use of leaf blade of *Macaranga tanarius* as a raw material for extraction is industrially advantageous from the standpoint of easiness of obtaining the raw material.

The raw material for extraction is subjected to an extraction procedure in the state when it is harvested, in the state that it is crushed, pulverized, or ground after the harvest, in the state that it is pulverized, crushed, or ground after the harvest and drying, or in the state that it is pulverized, crushed, or ground after the harvest and then is dried. In order to efficiently perform the extraction, the raw material for extraction is preferably crushed. The crushing of the raw material for extraction can be performed, for example, using a cutter, a shredder, or a crusher. The raw material for extraction can be pulverized using, for example, a mill, a crusher, or a grinder. The raw material for extraction can be ground using, for example, a kneader or a mortar.

The extraction solvent used for extracting a *Macaranga tanarius* extract from the raw material for extraction may be a solvent mixture of water and an organic solvent or may be an organic solvent such as lower alcohol, dimethyl sulfoxide, acetonitrile, acetone, ethyl acetate, hexane, glycerin, or propylene glycol. Examples of the lower alcohol that can be used include methanol, ethanol, propanol, isopropanol, and butanol. As the organic solvent, only one type of solvent may be used, or a mixture of a plurality of types of solvents may be used. When a solvent mixture of water and an organic solvent is used as the extraction solvent, the content of the organic solvent in the solvent mixture is preferably 50% by volume or more and more preferably 80% by volume or more. When the content of the organic solvent in a solvent mixture is 50% by volume or more, the active ingredient contained in *Macaranga tanarius* can be particularly efficiently extracted. The organic solvent is preferably lower alcohol and more preferably ethanol.

In the extraction solvent, for example, an organic salt, an inorganic salt, a buffer, an emulsifier, and dextrin may be dissolved.

The extraction is performed by immersing the raw material for extraction in the above extraction solvent for a predetermined time. In the extraction, according to need, for example, either stirring or heating or the both of them may be conducted for increasing the extraction efficiency. Furthermore, in order to minimize extraction of unnecessary impurities into the extraction solvent, prior to the extraction with the extraction solvent, the raw material for extraction may be prepared by being subjected to extraction with water or hot water and removing the extraction water in advance. The ingredient that is contained in *Macaranga tanarius* and presumably has an anti-MRSA activity is nymphaeols. The nymphaeols are water-insoluble. Impurities other than the nymphaeols are efficiently transferred to extraction water by boiling *Macaranga tanarius* with, for example, hot water and are thereby removed.

*A Macaranga tanarius* extract extracted from the raw material for extraction is subjected to solid liquid separation to separate and remove the residue of the raw material for extraction. The solid liquid separation is performed, for example, by a known method such as filtration or centrifugation. The *Macaranga tanarius* extract in a liquid form after the solid liquid separation may be concentrated according to need.

*A Macaranga tanarius* extract in a solid form can be obtained by removing the extraction solvent contained in the *Macaranga tanarius* extract in the liquid form, according to need. The removal of the extraction solvent from the *Macaranga tanarius* extract in the liquid form may be performed, for example, by heating under reduced pressure or by lyophilization.

The *Macaranga tanarius* extract extracted from *Macaranga tanarius* with an extraction solvent including at least an organic solvent contains at least one selected from nymphaeol-A (also known as 5,7,3',4'-tetrahydroxy-6-geranylflavanone), nymphaeol-B (also known as 5,7,3',4'-tetrahydroxy-2'-geranylflavanone), and nymphaeol-C (also known as 5,7,3',4'-tetrahydroxy-6-(3"',3"'-dimethylallyl)-2'-geranylflavanone). A main ingredient of the *Macaranga tanarius* extract is at least one selected from nymphaeol-A, nymphaeol-B, and nymphaeol-C, that is, nymphaeols, and the nymphaeols presumably have an anti-MRSA activity.

The *Macaranga tanarius* extract further contains propolin A (also known as 5,7,3',4'-tetrahydroxy-2'-(7"-hydroxy-3",7"-dimethyl-2"-octenyl)-flavanone). Furthermore, the *Macaranga tanarius* extract contains as minor ingredients, for example, 5,7,3',4'-tetrahydroxy-5'-geranylflavanone (also known as isonymphaeof-B), 5,7,3',4'-tetrahydroxy-5'-(7"-hydroxy-3",7"-dimethyl-2"-octenyl)-flavanone, 5,7,3',4'-tetrahydroxy-6-(7"-hydroxy-3",7"-dimethyl-2"-octenyl)-flavanone, 5,7,4'-trihydroxy-3'-(7"-hydroxy-3",7"-dimethyl-2"-octenyl)-flavanone, and 5,7,4'-trihydroxy-3'-geranylflavanone.

Among extract solutions each extracted from portions of *Macaranga tanarius,* an extract solution extracted from flowers, seeds, and fruit (containing wax) particularly contains high concentrations of nymphaeol-A, B, and C and isonymphaeol-B.

The anti-MRSA agent may contain a component other than the *Macaranga tanarius* extract as long as the anti-MRSA activity is not impaired. Examples of the component that can be contained in the anti-MRSA agent, in addition to the *Macaranga tanarius* extract, include an excipient, a base, an emulsifier, a stabilizer, and a flavoring.

The anti-MRSA agent may be in a liquid form or in a solid form. The dosage form of the anti-MRSA agent is not particularly limited and may be, for example, a powder, a dust, a granule, a tablet, a capsule, a pill, or a liquid.

The anti-MRSA agent can be used as, for example, a pharmaceutical product, a quasi drug, and a cleaning agent. MRSA is known to be a bacterial cause of nosocomial infection, and MRSA infections often occur particularly in patients with reduced resistance and the elderly. Accordingly, the anti-MRSA agent is preferably applied to, for example, a medical product, a medical device, an interior material for a hospital, an air inlet and an air outlet of a clean room in a hospital, and an interior material for a facility for the elderly. In the above cases, the anti-MRSA agent may be added, for example, to a molding material or a paint.

The anti-MRSA agent is desirably used in such a way that the total concentration of nymphaeol-A, nymphaeol-B, and nymphaeol-C, that is, the concentration of nymphaeols, at a site that could be a source of MRSA infection is preferably 25 ppm or more. When the concentration of nymphaeols is 25 ppm or more, an inhibitory action on the growth of MRSA is particularly well exerted.

The first embodiment has the following advantages.

The anti-MRSA agent of the present embodiment is a novel anti-MRSA agent containing a *Macaranga tanarius* extract as an active ingredient, and can be used for prevention of an MRSA infection, which is caused by the growth of MRSA.

In addition, since *Macaranga tanarius* grows significantly fast compared to other trees and can grow on degraded lands, the cultivation does not take much effort. Furthermore, since the *Macaranga tanarius* extract is originated from a plant, it is highly safe. Therefore, the anti-MRSA agent of the present embodiment is also excellent in stable supply of raw material, productivity, and safety.

### Second Embodiment

An anti-VRE agent of a second embodiment of the present invention will be described, focusing on differences from the anti-MRSA agent of the above-mentioned first embodiment.

Similar to the anti-MRSA agent, an anti-VRE agent of the second embodiment contains as an active ingredient an Oobagi extract extracted from Oobagi with an extraction solvent including at least an organic solvent.

The anti-VRE agent may contain a component other than the *Macaranga tanarius* extract as long as the anti-VRE activity is not impaired. Examples of the component that can be contained in the anti-VRE agent, in addition to the *Macaranga tanarius* extract, include an excipient, a base, an emulsifier, a stabilizer, and a flavoring.

The anti-VRE agent may be in a liquid form or in a solid form. The dosage form of the anti-VRE agent is not particularly limited and may be, for example, a powder, a dust, a granule, a tablet, a capsule, a pill, or a liquid.

The anti-VRE agent can be used as, for example, a pharmaceutical product, a quasi drug, and a cleaning agent. Like MRSA, VRE is known to be a bacterial cause of nosocomial infection, and VRE infections often occur particularly in patients with reduced resistance and the elderly. Accordingly, the anti-VRE agent is preferably applied to, for example, a medical product, a medical device, an interior material for a hospital, an air inlet and an air outlet of a clean room in a hospital, and an interior material for a facility for the elderly. In the above cases, the anti-VRE agent may be added, for example, to a molding material or a paint.

The anti-VRE agent is desirably used in such a way that the total concentration of nymphaeol-A, nymphaeol-B, and nymphaeol-C, that is, the concentration of nymphaeols, in a site that could be a source of VRE infection is preferably 8 ppm or more. When the concentration of nymphaeols is 8 ppm or more, an inhibitory action on the growth of VRE is particularly well exerted.

The second embodiment has the following advantages.

The anti-VRE agent of the present embodiment is a novel anti-VRE agent containing a *Macaranga tanarius* extract as an active ingredient, and can be used for prevention of a VRE infection, which is caused by the growth of VRE.

In addition, since *Macaranga tanarius* grows significantly fast compared to other trees and can grow on degraded lands, the cultivation does not take much effort. Furthermore, since the *Macaranga tanarius* extract is originated from a plant, it is highly safe. Therefore, the anti-VRE agent of the present embodiment is also excellent in stable supply of raw material, productivity, and safety.

The above-described embodiments may be modified as follows.

The anti-MRSA agent and the anti-VRE agent of the above embodiments may contain at least one selected from nymphaeol-A, nymphaeol-B, and nymphaeol-C that are not originated from *Macaranga tanarius* extracts, as an active ingredient, instead of the *Macaranga tanarius* extract or in addition to the *Macaranga tanarius* extract. Nymphaeol-A, nymphaeol-B, and nymphaeol-C that are not originated from *Macaranga tanarius* extracts can be obtained by, for example, chemical synthesis.

Next, the present invention will be further specifically described with reference to examples.

### Example 1

### <Preparation 1 of Macaranga tanarius extract>

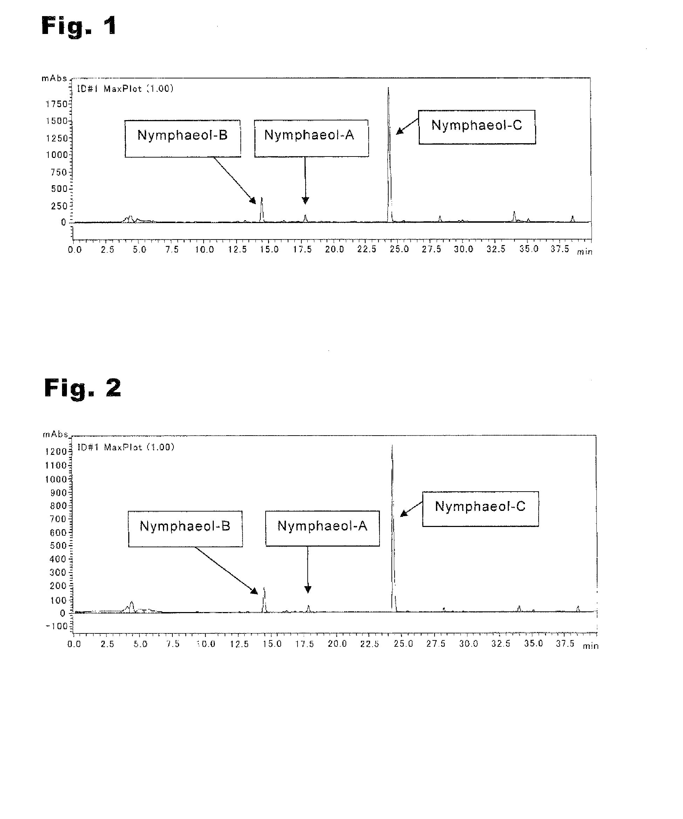

Frozen raw leaves of *Macaranga tanarius* harvested in Okinawa were thawed, and the leaves were cut into small pieces with scissors. Thirty grams of the cut raw leaves were immersed in 100 mL of a solvent mixture consisting of 90 parts by volume of ethanol and 10 parts by volume of water and left standing at room temperature for two weeks, followed by filtration to yield the filtrate as a *Macaranga tanarius* extract solution. The *Macaranga tanarius* extract solution was lyophilized to prepare a *Macaranga tanarius* extract that was a powder of the solid content contained in the *Macaranga tanarius* extract solution. The total concentration of nymphaeol-A, nymphaeol-B, and nymphaeol-C, that is, the concentration of nymphaeols, in the *Macaranga tanarius* extract in the powder form was 50% by mass when calculated from the chromatogram shown in Fig. 1 obtained by analyzing the *Macaranga tanarius* extract under the following HPLC conditions.

### HPLC conditions

System: PDA-HPLC system (Shimadzu Corp.), LC10ADvp series, UV: SPD-10Avp, PDA: SPD-M10Avp,
Column: Luna C18 (2 × 250 mm) (Shimadzu GLC),
Solvent: A: water (5% acetic acid), B: acetonitrile (5% acetic acid)

### Dissolution condition:

0 to 20 minutes
(gradient dissolution : A:B = 80:20 → A:B = 30:70)
20 to 50 minutes
(gradient dissolution : A:B = 30:70 → A:B = 0:100)
50 to 60 minutes (A:B = 0:100)
60 to 75 minutes (A:B = 80:20)
Flow rate: 0.2 mL/min
PDA detection: UV from 190 to 370 nm
UV detection: UV 287 nm
Injection amount: 20 µL
Temperature: 40°C

### <Test of antimicrobial activity of Macaranga tanarius extract on MRSA>

An MRSA bacterial strain, Methicillin-resistant *Staphylococcus aureus* ATCC 33591 strain, was inoculated in a *Staphylococcus* No. 110 agar plate medium (manufactured by Nippon Bio-Supply Center) using a platinum loop, and subsequently cultured at 37°C for 48 hours.

A colony of the MRSA strain proliferated by culturing was collected from the medium using a platinum loop and dissolved in 1 mL of physiological saline, and diluted with sterilized PBS and a Mueller-Hinton liquid medium (manufactured by Nippon Bio-Supply Center) to prepare bacterial liquids for inoculation having bacterial counts of 1 × 10⁴, 1 × 10⁶, and 1 × 10⁸ cfu/mL.

The *Macaranga tanarius* extract obtained in the above-mentioned "Preparation 1 of *Macaranga tanarius* extract" was diluted with a Mueller-Hinton broth medium (manufactured by Difco Laboratories, Inc.) to prepare sample media having respective final concentrations of the *Macaranga tanarius* extract of 0.005% by mass, 0.01% by mass, 0.05% by mass, and 0.2% by mass. A control medium having a final concentration of the *Macaranga tanarius* extract of 0% by mass was also prepared using a Mueller-Hinton broth medium.

To 1 mL of each of the sample media and the control medium, 10 µL of the bacterial liquid for inoculation was inoculated and subjected to static culture at 37°C for 20 hours. Subsequently, 0.1 mL of the cultured product was collected from each medium and smeared on *Staphylococcus* No. 110 agar plate media (manufactured by Nippon Bio-Supply Center). The bacterial strain on each plate medium was then cultured at 37°C for 48 hours. The results of determining the presence or absence of a colony on each plate medium after culturing are shown in Table 1. In the column titled "Degree of sensitivity" in Table 1, "+" indicates that a colony was found, and "-" indicates that no colony was found.

**Table 1**

| Concentration of *Macaranga tanarius* extract (% by mass) | Degree of sensitivity | | |
|---|---|---|---|
| | 1×10⁴ (cfu/mL) | 1×10⁶ (cfu/mL) | 1×10⁸ (cfu/mL) |
| 0 | + | + | + |
| 0.005 | - | - | - |
| 0.01 | - | - | - |
| 0.05 | - | - | - |
| 0.2 | - | - | - |

As shown in Table 1, it was observed that the growth of MRSA was completely inhibited when the concentration of the *Macaranga tanarius* extract was 0.005% by mass or more, which was 0,0025% by mass (25 ppm) or more when converted to the concentration of nymphaeols.

### <Test of antimicrobial activity of Macaranga tanarius extract on VRE>

A VRE bacteria! strain, *Enterococcus faecium* NCTC 12204 strain, was inoculated in a Mueller-Hinton broth medium (manufactured by Difco Laboratories, Inc.) and cultured at 37°C for 18 to 20 hours. Subsequently, a bacterial liquid for inoculation having a bacterial count of 1 × 10⁶ cfu/mL was prepared.

The *Macaranga tanarius* extract obtained in the above-mentioned "Preparation 1 of *Macaranga tanarius* extract" was diluted with ethanol to prepare sample liquids in a doubling-dilution series. That is, sample liquids having respective concentrations of *Macaranga tanarius* extract of 10.0% by mass (10.00 × 10⁴ ppm), 5.00% by mass (5.00 × 10⁴ ppm), 2.50% by mass (2.50 × 10⁴ ppm), 1.25% by mass (1.25 × 10⁴ ppm), 0.625% by mass (0.625 × 10⁴ ppm), 0.313% by mass (0.313 × 10⁴ ppm), and 0.0156% by mass (0.0156 × 10⁴ ppm) were prepared.

To 99% by mass of Mueller-Hinton agar media (manufactured by Difco Laboratories, Inc.), which had been kept warm at 50 to 60°C, 1 % by mass of each sample liquid was added and thoroughly mixed. The mixtures were then dispensed into petri dishes and solidified. Accordingly, plate media for sensitivity measurement having respective concentrations of *Macaranga tanarius* extract of 1,000 ppm, 500 ppm, 250 ppm, 125 ppm, 62.5 ppm, 31.3 ppm, and 15.6 ppm were prepared.

The bacterial liquid for inoculation was streaked (approximately 1 to 2 cm) on each plate medium for sensitivity measurement using a loop made of resin. Subsequently, the bacterial strain on each plate medium was cultured at 37°C for 18 to 20 hours.

Observation of the plate media after culturing showed that the growth of VRE was inhibited in the plate media having a concentration of the *Macaranga tanarius* extract of 15.6 ppm or more. In other words, the minimum inhibitory concentration (MIC) of the *Macaranga tanarius* extract against VRE was 15.6 ppm. As described above, since the *Macaranga tanarius* extract of Example 1 contains 50% by mass of nymphaeols, it is speculated from the above results that the minimum inhibitory concentration of nymphaeols against VRE was 7.8 ppm.

### Example 2

### <Preparation 2 of Macaranga tanarius extract>

Thirty grams of cut raw leaves of *Macaranga tanarius* were immersed in 95°C water for 30 minutes. The water was removed by filtration, and the remaining leaves were immersed in 100% ethanol for 3 days, followed by filtration to yield the filtrate as a *Macaranga tanarius* extract solution. The *Macaranga tanarius* extract solution was lyophilized to prepare a *Macaranga tanarius* extract that was a powder of the solid content contained in the *Macaranga tanarius* extract solution. The total concentration of nymphaeol-A, nymphaeol-B, and nymphaeol-C, that is, the concentration of nymphaeols, in the *Macaranga tanarius* extract in the powder form was 40% by mass when calculated from the chromatogram shown in Fig. 2 obtained by analyzing the *Macaranga tanarius* extract under the above-mentioned HPLC conditions.

### <Test of antimicrobial activity of Macaranga tanarius extract on MRSA and VRE>

An antimicrobial activity test was performed as in Example 1. Regarding the *Macaranga tanarius* extract prepared in Example 2, similar results as those of the *Macaranga tanarius* extract prepared in Example 1 were obtained to show that the antimicrobial activities of the *Macaranga tanarius* extract prepared in Example 1 and the *Macaranga tanarius* extract prepared in Example 2 were similar to each other.

## Claims

1. An anti-methicillin-resistant *Staphylococcus aureus* agent **characterized by** comprising as an active ingredient a *Macaranga tanarius* extract extracted from *Macaranga tanarius* with an extraction solvent including at least an organic solvent.

2. An anti-methicillin-resistant *Staphylococcus aureus* agent **characterized by** comprising as an active ingredient at least one selected from nymphaeol-A, nymphaeol-B, and nymphaeol-C.

3. The anti-methicillin-resistant *Staphylococcus aureus* agent according to claim 2, wherein the at least one selected from nymphaeol-A, nymphaeol-B, and nymphaeol-C is originated from an extract of *Macaranga tanarius.*

4. An anti-vancomycin-resistant *Enterococcus* agent **characterized by** comprising as an active ingredient a *Macaranga tanarius* extract extracted from *Macaranga tanarius* with an extraction solvent including at least an organic solvent.

5. An anti-vancomycin-resistant *Enterococcus* agent **characterized by** comprising as an active ingredient at least one selected from nymphaeol-A, nymphaeol-B, and nymphaeol-C.

6. The anti-vancomycin-resistant *Enterococcus* agent according to claim 5, wherein the at least one selected from nymphaeol-A, nymphaeol-B, and nymphaeol-C is originated from an extract of *Macaranga tanarius.*
